# EUROPEAN PATENT APPLICATION

(11) **EP 1 138 276 A1**
(43) Date of publication of application: **04.10.2001**
(21) Application number: 00106701.6
(22) Date of filing: 29.03.2000
(51) Int. Cl.: A61C 19/00

(54) **Dental material curing apparatus**

(71) Applicant: CMS-Dental ApS, 1408 Kobenhavn K (DK)
(72) Inventor: Kert, Jimmie, 3500 Vaerloese (DK)
(74) Representative: Roerboel, Leif

(57) **Abstract**

An object of the present invention is to provide an apparatus for curing light curable materials intended to allow shorter curing times than have hitherto been practicable.

The apparatus provided by the invention for curing photocurable materials, comprises a housing (1), a light source, a light guide (3) extending at least partially out of the housing (1) and a light concentrator element (7) of light conducting material having substantially frusto conical shape and having a light input end (7b) for receiving light from the light source and having a light output end (7a) through which a concentrated beam of light leaves the light concentrator element (7). The light source comprises an array of light emitting diodes (20) mounted on a print board (22) facing the light input 7b of the light concentrator element 7.

## Description

### TECHNICAL FIELD

The present invention relates to a dental material curing apparatus as set forth in the preamble of claims 1 and 2.

### BACKGROUND ART

Light cured dental materials are widely used in modern dentistry. The clinical performance of a light curing composite restoration is greatly influenced by the quality of the curing light. Only those wavelengths strongly absorbed by the photoinitiator are effective for photopolymerization. The absorption curve of camphoroquinone, which is the most common photoinitiator, lies between 410 and 500 nm, with its maximum at 470 nm. The intensity of the light source is critical because a threshold radiation is required to cause polymerisation at a specific depth during a specific exposure time. Due to the presence of inhibitors, at a specific depth, polymerisation does not occur until all of the inhibitor (at that depth) has been consumed by the radicals generated in the immediate vicinity.

Any light cured material will shrink after curing. Therefore it is recommended that dentists build up restorations in layers not thicker than 2-3 mm each, thus reducing the overall shrinkage. During the curing process, the materials must be kept dry without contact with saliva and blood. It is therefore not surprising that practising dentists are interested in as short curing time of the material as possible.

Traditionally halogen light bulbs have been used to cure dental materials. A curing apparatus of this kind is disclosed in US-A-5,803,729. Typically such apparatuses comprise a housing, a halogen bulb with build-in reflector, and a filter between the bulb and curved light guide extending out of the housing. A cooling system and a microprocessor controlled power supply are also common.

The most important drawback of using halogen light sources is that only a small fraction of the light that they emit can actually cure the dental material, as these light sources emit light in a broad spectrum from the UV to infrared. High quality filters must be used to filter the unwanted light to avoid excessive heating of the material and tooth structure. Due to these drawbacks the conventional halogen light bulb apparatuses need to be applied about 40 seconds to secure a complete polymerisation. This is a long time for a dentist since the material to be cured has to be kept dry and free from saliva and blood. Thus there is a need for an apparatus that allows for faster curing.

### DISCLOSURE OF THE INVENTION

On this background, it is the object of the present invention to provide a curing apparatus of the kind referred to initially, which overcomes the above-mentioned problems. This object is achieved in accordance with claim 1 and 2 by including an array of light emitting diodes in the light source. By concentrating the light of an array of light emitting diodes an apparatus is provided that emits light in a relatively narrow band of the wavelength spectrum corresponding to those wavelengths strongly absorbed by the photoinitiator. Thus the apparatus allows to apply significantly higher power in effective part of the spectrum without excessive heating of the material to be cured and the tooth structure. Consequently the curing time can be reduced to roughly 9 seconds with the apparatus according to the present invention.

According to an embodiment of the invention the light source further comprises a halogen light bulb and a reflector arrangement.

According to another embodiment of the invention the apparatus comprises a second light guide having a light receiving end for receiving light from the light bulb and reflector arrangement and a light output end directing the light to the light input end of the light concentrator.

According to another embodiment of the invention the apparatus comprises a number of said light concentrator elements in series, preferably provided with interconnecting light guides.

### BRIEF DESCRIPTION OF THE DRAWINGS

In the following detailed portion of the present description, the invention will be explained in more detail with reference to the exemplary embodiments shown in the drawings, in which
Figure 1 is a sectional view of a preferred embodiment of the apparatus according to the invention,
Figure 2 is a detail of the apparatus according to another embodiment of the invention,
Figure 3 is a detail in of a further embodiment of the invention,
Figure 4 is a detail of a last embodiment of the invention, and
Figure 5 is another view on the detail of the last embodiment of the invention.

### DETAILED DESCRIPTION OF THE INVENTION

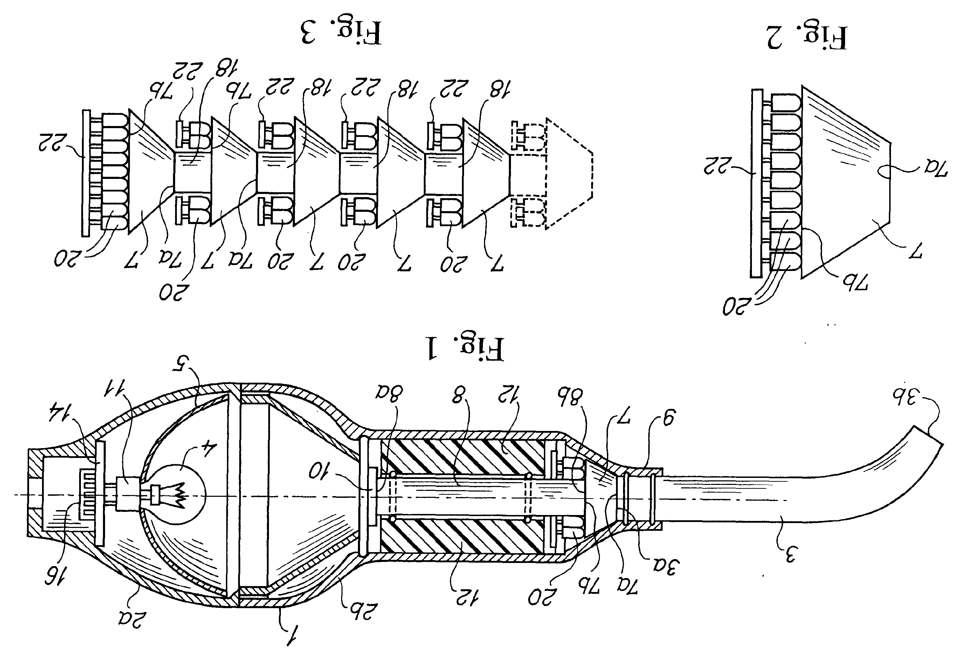

The apparatus according to a preferred embodiment of the invention shown in FIG. 1 for curing dental filling material includes a housing 1. The housing 1 comprises a dome-shape rear part 2a and a tapered front part 2b which together define an enclosure. A handle (not shown) may be provided to allow the curing light to be held and manipulated by the dentist in use. A first light guide 3 protrudes from the forward end of the housing 1. Within the tapered front part 2b of the housing 1 is mounted a second light guide 8 and a non-imaging light concentrator 7.

Within the dome-shaped rear part 2a of the housing 1 is an arrangement comprising a halogen bulb 4 and a reflector 5. The bulb 4 is a conventional halogen bulb and is mounted at the focal point of the reflector. The reflector is positioned to direct the light forwardly toward a second rod-shaped light guide 8. Bulb 4 is plugged into a socket 11 which is supported by a print board 14 with a microprocessor 16. A cooler (not shown) may be placed behind the bulb 4 and reflector 5 arrangement. The microprocessor 16 shall monitors the internal temperature of the apparatus, and cuts the power to the bulb 4, if necessary, to avoid overheating of the apparatus. The microprocessor 16 further monitors if the halogen bulb 4 ceases to work and thus issues an alarm signal. Moreover the microprocessor 16 can cut off the power to the bulb 4 after a pre-set delay.

The Bulb 4 and the reflector 5 are disposed symmetrically respect to a longitudinal axis A--A of the housing 1. A sleeve 9 at the tapered front part 2b of the housing receives the first light guide 3 and is also symmetrical about axis A--A so that the first light guide 3 itself will be centered on that axis. The fist light guide 3 has an input end 3a for receiving light from the light source, and an output end 3b for directing light to the target material to be cured. Examples of suitable materials for the first light guide 3 are glass, polycarbonate, and polymethylmethacrylate.

A light concentrator element 7 of frusto-conical shape is arranged between the light guide 3 and the second light guide 8. The base "input" surface 7b of the light concentrator element 7 has a diameter which is larger that the diameter of the output end 8b of the second light concentrator 8 thus allowing a number of light emitting diodes (LEDs) 20 to be placed circular around the glass rod. Typically 10 to 20 laser diodes or blue LEDs are used. The LEDs 20 are mounted on a print board 22 facing the light input end 7b of the light concentrator element 7, and angled such as to optimise the transmittance of the LED light through the light concentrator 7 and into the first light guide 3 which functions as a standard dental glass multimode fibre optical tip.

The output end 7a of the light concentrator has a diameter corresponding to the diameter of the input end 3a of the first light guide 3. The first light guide 3 is preferably tapered. The second light guide 8 has approximately a diameter corresponding to the diameter of the input end 3a of the first light guide 3.
The first light guide 3 is easily removable for cleaning or replacement.

According to the preferred embodiment the light concentrator element 7 and the second light guide 8 are formed of one piece of material. The concentrator element 7 and the second light guide 8 are positioned symmetrically about axis A--A with the input end 8a of the second light guide 8 facing the light bulb 4 and reflector 5 arrangement for receiving light from this source, and the narrower (output) end 7a of the light concentrator element 7 in "line" with the first light guide 3. The light concentrator element 7 and the second light guide 8 may be made of glass, or an appropriate plastic material such a polycarbonate, or polymethylmethacrylate, in quality that ensures a minimum of loss of light.

At the input end 8a of the second light guide 8 is an interferential filter 10 of the type conventionally used in curing apparatuses, with a band of transmittance (400-500 nm). Inside the front part 2b is arranged a generally tube shaped plastic support element 12 receiving and holding the light concentrator element 7, the second light guide 8 and the filter 10 in place.

The light rays from the bulb 4 are directed by the reflector 5 forwardly through the filter 10 into the second light guide 8, pass the light concentrator 7 and then delivered into the first light guide 3. The light from the LEDs 20 enters the light concentrator element 7 and mixes with the light from the halogen bulb 4. The light from the halogen bulb 4 will be characterised by the window of the filter, typically allowing only light in the spectrum of 400 to 500 nm. In that spectrum the intensity of each wavelength will be in the same magnitude. The light from the LEDs will have a typically wavelength of 470 nm +/- 5 nm. At the optimal wavelengths the light from the LEDs will have about the same or higher intensity, compared to the light originating from the halogen bulb 4. Therefore the combined intensity of the light with a wavelength of around 470 nm leaving the output end 3b of the fist light guide, will be about double as high as achieved with either one of the light sources alone.

The concentrator element 7 may be made from different materials (e.g. polymethylmethacrylate, polycarbonate or glass). Modified shapes including exponential and stepped cones may also be used.

The sum of light output of the diodes 20 is between 5 to 20mW. Due to the exact match of the wavelength of the light to the photoinitiator this part of the light stands for a substantial part of the curing effect. The halogen bulb has a power consumption of about 75 Watt. Due to the fact that only a minor part of this light is in the effective part of the spectrum this large energy input stands for the remaining of the curing effect.

FIG. 2 illustrates an alternative embodiment of the invention which uses exclusively light emitting diodes as a light source. The input end 7b of the light concentrator 7 is completely used for receiving light from the LEDs. This embodiment allows using a much more simple power supply than needed for a powerful halogen lamp. Due to the absence of excess heat there is no need for a cooling arrangement. Depending on the quality of the LEDs, the size and quality of the light concentrator element the curing time for this embodiment will be about 10 to 20 seconds.

FIG. 3 shows another alternative embodiment of the invention which uses exclusively light emitting diodes as a light source. A number of cone shaped light concentrator elements 7 are arranged in series. The light concentrator elements 7 can simply be stacked to form a string (not shown) or can be provided with intimidate light guides 18. LEDs are mounted at the light input end 7b of each light concentrator 7.

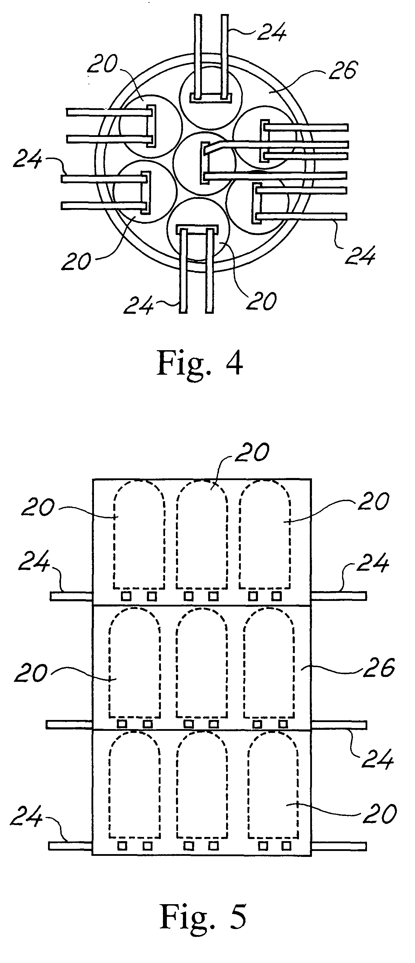

FIGS. 4 and 5 show a further embodiment of the invention which does not use a light concentrator element. Instead the diodes are arranged in a solid light-conducting element 26 which is preferably made of the same material as the LED body. Thus the LEDs 20 can be arranged one behind another with only a small amount of the emitted light being lost. Preferably seven LEDs 20 are arranged in one layer with 3 layers of LEDs 20 arranged one behind another. The pins 24 of the LEDs 20 are bend sideways so that they extend laterally from the solid light conducting element 26 for connection to source of electric power (not shown).

Several of such solid light conducting 26 elements containing LEDs 20 may be arranged in series to obtain a higher output effect.

This embodiment can also be combined with a halogen bulb 4 and a second light guide 8; as a matter of fact the light-conducting element 26 can be integral with the second light guide 8.

### LIST OF REFERENCE NUMERALS

- 1: Housing
- 2a: Rear part
- 2b: Front part
- 3: First light guide
- 3a: Input end
- 3b: Output end
- 4: Halogen bulb
- 5: Reflector
- 7: Light concentrator element
- 7a: Output end
- 7b: Input end
- 8: Second light guide
- 8a: Input end
- 8b: Output end
- 9: Sleeve
- 10: Filter
- 11: Socket
- 12: Plastic support element
- 14: Print board
- 16: Micro processor
- 18: Intermediate light guide
- 20: Light emitting diode (LED)
- 22: Print board
- 24: LED pin
- 26: Solid light conducting element

## Claims

1. Apparatus for curing photocurable materials, comprising
a housing (1), a light source, a light guide (3) extending at least partially out of the housing (1) and a light concentrator element (7) of light conducting material having substantially frusto conical shape and having a light input end (7b) for receiving light from the light source and having a light output end (7a) through which a concentrated beam of light leaves the light concentrator element (7),
**characterised in that** the light source comprises an array of light emitting diodes (20) arranged at the light input end (7b) of the light concentrator element (7).

2. Apparatus for curing photocurable materials, comprising
a housing (1), a light source, a light guide (3) extending at least partially out of the housing (1), **characterised in that** the light source comprises an array of light emitting diodes (20) arranged in a solid light conducting element.

3. Apparatus according to claim 2, **characterised in that** a number of the light emitting diodes (20) are placed behind one another.

4. Apparatus according to any of claims 1 to 3 **characterised by** the light source further comprising a light bulb (4) and a reflector (5) arrangement, whereby the light bulb is preferably a halogen bulb.

5. Apparatus according to claim 4, **characterised by** comprising a second light guide (8) having a light receiving end (8a) for receiving light from the light bulb (4) and reflector (5) arrangement and a light output end (8b) directing the light to the light input end (7b) of the solid light conducting element.

6. Apparatus according to claim 4, **characterised by** comprising a second light guide (8) having a light receiving end (8a) for receiving light from the light bulb (4) and reflector (5) arrangement and a light output end (8b) directing the light to the light input end (7b) of the light concentrator element (7).

7. Apparatus according to claim 6, **characterised in that** the light concentrator element (7) and the second light guide (8) are formed of one singe piece of light conducting material.

8. Apparatus according to claim 5 or 6 **characterised in that** the light emitting diodes (20) are placed around the second light guide (8) and adjacent to the light input (7b) end of the light concentrator element (7).

9. Apparatus according to any of claims 1 to 8 **characterised by** comprising a number of said light concentrator elements (7) in series, preferably provided with interconnecting light guides (18).

10. Apparatus according to any of the claims 1 to 9, **characterised in that** the light emitting diodes (20) are laser diodes producing light in a with wavelength band of 470 +/- 15 nm.

11. Apparatus according to any of claims 4 to 10 **characterised in that** it comprises a spectral filter (10) between the light bulb and the light concentrator, preferably a spectral filter (10) that is transparent only to light with a wavelength range of 400 to 500 nm.
